# EUROPEAN PATENT APPLICATION

(11) **EP 4 840 398 A1**
(43) Date of publication of application: **18.11.2026**
(21) Application number: 26174180.5
(22) Date of filing: 23.04.2026
(51) Int. Cl.: A61B 5/18, A61B 5/024, A61B 5/0531, B60W 40/08, A61B 5/00

(54) **STEERING WHEEL HANDS-ON DETECTION THROUGH ECG SIGNAL VERIFICATION**

(30) Priority: 13.05.2025 US 202519206980
(71) Applicant: STMicroelectronics International N.V., 1228 Plan-les-Ouates, Geneva (CH)
(72) Inventor: ALESSI, Enrico Rosario, 20864 Agrate Brianza (MB) (IT); PASSANITI, Fabio, 20864 Agrate Brianza (MB) (IT)
(74) Representative: Studio Torta S.p.A.

(57) **Abstract**

According to an embodiment, a hands-on detection system includes a plurality of electrodes positioned on a steering wheel, an analog front-end circuit coupled to the electrodes, and a controller. The analog front-end circuit transmits signals through stimulus electrodes, receives signals through receiving electrodes when hands contact the wheel, provides reference voltages to reference electrodes for local polarization, and detects biopotential signals between electrodes. The controller verifies physical contact by analyzing received signals and verifies physiological characteristics by analyzing biopotential signals. The system employs a dual verification approach, first confirming physical contact through electrostatic charge variation detection and then validating proper hand placement through electrocardiogram signal analysis.

## Description

### TECHNICAL FIELD

The present disclosure generally relates to electronic devices and, in particular embodiments, to steering wheel hands-on detection through ECG signal verification.

### BACKGROUND

Vehicle steering systems serve as the primary interface between drivers and their vehicles, enabling directional control through manual input. Steering wheels in modern vehicles function as control mechanisms and platforms for various driver interaction technologies. As vehicles incorporate increasingly sophisticated assistance features, the steering wheel has evolved to accommodate additional functionalities beyond basic directional control.

This evolution of steering systems reflects the changing relationship between drivers and vehicles. Driver engagement with steering systems remains essential for safe vehicle operation across various levels of automation. The physical contact between a driver's hands and the steering wheel represents a fundamental aspect of vehicle control, providing tactile feedback about road conditions and vehicle response. This physical connection between driver and vehicle has traditionally served as an indicator of driver attentiveness and readiness to control the vehicle.

Beyond mechanical control, the steering wheel's position at the center of driver interaction makes it an ideal location for monitoring driver state. Physiological monitoring in automotive environments has emerged as an area of interest in transportation safety. Collecting and analyzing biometric data during vehicle operation can provide insights into driver states, including alertness and stress levels. Electrocardiogram (ECG) signals, which measure the electrical activity of the heart, represent one form of biometric data that can be collected in a vehicle environment under appropriate conditions.

This monitoring capability has become particularly relevant with the rise of automation in vehicles. Advanced Driver-Assistance Systems (ADAS) and autonomous driving technologies have transformed modern vehicles by automating certain driving tasks to enhance safety and convenience. These systems can include, for example, lane-centering assistance, adaptive cruise control, and collision avoidance. While these technologies can reduce driver workload, they typically require ongoing driver supervision and readiness to intervene. Consequently, vehicles equipped with such systems often incorporate mechanisms to verify driver engagement, particularly through monitoring hand contact with the steering wheel or visual attention to the road. These monitoring systems help ensure drivers remain attentive and prepared to resume manual control when necessary, even as the vehicle performs certain driving tasks autonomously.

Supporting these advanced functions requires electronics integrated throughout the vehicle. The integration of electronic systems within steering components has expanded the capabilities of modern vehicles. These systems can include various sensors and processing elements that enhance vehicle functionality while maintaining the familiar steering wheel interface that drivers have used for generations. The steering wheel's consistent presence in the driver's immediate environment makes it a natural location for incorporating additional sensing capabilities.

As these technologies mature, they must conform to evolving safety standards. Automotive safety standards continue to evolve alongside technological advancements, with regulatory frameworks establishing requirements for various vehicle systems. These standards help ensure that new technologies support rather than detract from safe vehicle operation. As vehicles incorporate more advanced driver assistance features, the interaction between driver and vehicle systems becomes increasingly important to overall safety.

### SUMMARY

The present invention relates to a system and a method for hands-on detection as defined in the annexed claims.

Technical advantages are generally achieved by embodiments of this disclosure, which describe, in general terms, steering wheel hands-on detection through ECG signal verification.

A first aspect relates to a system for hands-on detection, the system comprising a plurality of electrodes positioned on a steering wheel; an analog front-end circuit coupled to the plurality of electrodes, the analog front-end circuit configured to: transmit a signal through at least one of the plurality of electrodes, receive the signal through at least one other of the plurality of electrodes in response to hands being in contact with the steering wheel, provide a reference voltage to at least one of the plurality of electrodes, and detect biopotential signals between at least two of the plurality of electrodes; and a controller coupled to the analog front-end circuit, the controller configured to: verify physical contact with the steering wheel based on the received signal, analyze the biopotential signals to verify presence of physiological characteristics, and generate a response based on the analysis of the biopotential signals.

A second aspect relates to a method for hands-on detection on a steering wheel, the method comprising: transmitting a reference voltage pattern through stimulus electrodes positioned on the steering wheel; receiving an electrostatic charge variation signal through receiving electrodes positioned on the steering wheel in response to hands bridging the stimulus electrodes and the receiving electrodes; providing a reference voltage to a reference electrodes positioned adjacent to the receiving electrodes to establish local polarization; detecting electrocardiogram (ECG) signals between the receiving electrodes positioned on opposite sides of the steering wheel; verifying physical contact with the steering wheel by validating the voltage pattern through the electrostatic charge variation signal; analyzing the detected ECG signals to verify presence of physiological characteristics; and generating a response based on the analysis of the detected ECG signals.

A third aspect relates to a system, the system comprising a steering wheel with a plurality of electrode sets distributed around its circumference, each electrode set comprising a receiving electrode, a stimulus electrode, and a reference electrode; a vertical analog front end circuit coupled to the multiple electrode sets, the vertical analog front end circuit configured to: transmit reference voltage patterns through the stimulus electrodes, detect electrostatic charge variations through the receiving electrodes, and provide reference voltages to the reference electrodes for local polarization; and a controller coupled to the vertical analog front end circuit, the controller configured to scan the multiple electrode sets to detect hand contact at various positions around the steering wheel, analyze detected signals to verify presence of physiological characteristics, generate a response based on the analysis of the detected signals, monitor heart rate information derived from the detected signals, and correlate information on the heart rate with driving maneuvers detected through motion sensors.

Embodiments can be implemented in hardware, software, or any combination thereof.

### BRIEF DESCRIPTION OF THE DRAWINGS

For a more complete understanding of the present disclosure and the advantages thereof, reference is now made to the following descriptions taken in conjunction with the accompanying drawings, in which:
Figure 1 is a vehicle interior environment where a driver's hand is positioned on a steering wheel;
Figure 2 is a hands-on detection (HoD) system;
Figure 3 is an electrode configuration for hands-on detection;
Figure 4 is a flowchart of method for electrocardiogram (ECG) signal processing and morphology checking;
Figure 5 is a hands-on detection (HoD) system for operation without hands tracking;
Figure 6 is a hands-on detection (HoD) system for operation with hands-tracking capability;
Figure 7 is a method for operating the HoD system;
Figure 8 is a flowchart of a method for hands-on detection, heartbeat computation, and health assistance; and
Figure 9 is a controller system that can be implemented as controller in a hands-on detection system.

### DETAILED DESCRIPTION OF EMBODIMENTS OF THE INVENTION

This disclosure provides many applicable inventive concepts that can be embodied in a wide variety of specific contexts. The particular embodiments are merely illustrative of specific configurations and do not limit the scope of the claimed embodiments. Features from different embodiments can be combined to form further embodiments unless noted otherwise. Various embodiments are illustrated in the accompanying drawing figures, where identical components and elements are identified by the same reference number, and repetitive descriptions are omitted for brevity.

Variations or modifications described in one of the embodiments can also apply to others. Further, various changes, substitutions, and alterations can be made herein without departing from the spirit and scope of this disclosure as defined by the appended claims.

While the aspects described in this disclosure are presented primarily in the context of automotive steering wheels and driver monitoring systems, it should also be appreciated that these aspects can also apply to other control interfaces where user engagement detection is beneficial. In particular, aspects of this disclosure can similarly apply to aircraft control systems, industrial machinery operations, medical equipment interfaces, gaming controllers, or any human-machine interface where verifying proper hand contact and user physiological state can enhance safety or performance.

Embodiments of the disclosure provide systems and methods for detecting proper hand placement on a steering wheel using a signal code transmission and electrocardiogram (ECG) verification. The disclosed approach utilizes electrodes positioned on the steering wheel that serve dual purposes-detecting physical contact through signal transmission and receiving biopotential signals from the driver's hands. This dual verification methodology enhances reliability compared to conventional detection methods by confirming not only the presence of contact with the steering wheel but also verifying that both hands of the same person are properly positioned.

In embodiments, a disclosed system includes a vertical analog front end (VAFE) circuit coupled to multiple electrodes on the steering wheel. The VAFE circuit transmits code signals through transmitter electrodes and receives these signals through receiver electrodes when hands are properly positioned, providing initial verification of physical contact. The system employs local polarization techniques that establish reference voltages near the detection electrodes, reducing environmental electrical noise and improving signal quality for biopotential measurements.

An aspect of the disclosure involves analyzing ECG signals detected between electrodes positioned on opposite sides of the steering wheel. The system can detect valid ECG signals when both hands of the same person contact the electrodes. The system can apply signal processing algorithms to verify ECG morphology, analyzing parameters such as signal amplitude, peak width, and beat-to-beat intervals to confirm valid heart rhythm patterns.

Some embodiments incorporate multiple electrodes distributed around the steering wheel, enabling scanning operations to determine precise hand positions. This configuration allows the system to function regardless of where the driver places their hands and can detect specialized conditions such as crossed hands during turning maneuvers. Scanning provides flexibility for various driving styles while maintaining reliable detection performance.

The system integrates motion sensing capabilities through accelerometers and gyroscopes embedded in the same chip as the VAFE circuit. The motion sensors are fixed on the steering wheel, ensuring direct and accurate measurement of steering wheel movements. This integration allows correlation between steering wheel movements and ECG measurements, enabling context-aware detection that can adapt to different driving scenarios. For example, the system modifies its detection requirements during specific maneuvers like parking, when drivers typically need to temporarily remove one hand from the steering wheel.

Beyond hands-on detection, the system provides additional health monitoring capabilities (e.g., in-cabin sensing) by analyzing the collected ECG signals. The ECG waveforms contain features that can indicate various aspects of cardiac function, potentially allowing the system to detect signs of driver fatigue, stress, or other physiological states that might affect driving performance. This added functionality transforms the steering wheel from a simple control interface into a comprehensive driver monitoring platform.

The disclosed approach offers advantages in reliability, particularly in challenging conditions such as when drivers wear gloves or in the presence of moisture. Using code signal verification and ECG morphology confirmation, the system provides robust detection that resists false positives from objects placed on the steering wheel or environmental factors that might confuse simpler detection methods. These and additional details are further discussed below.

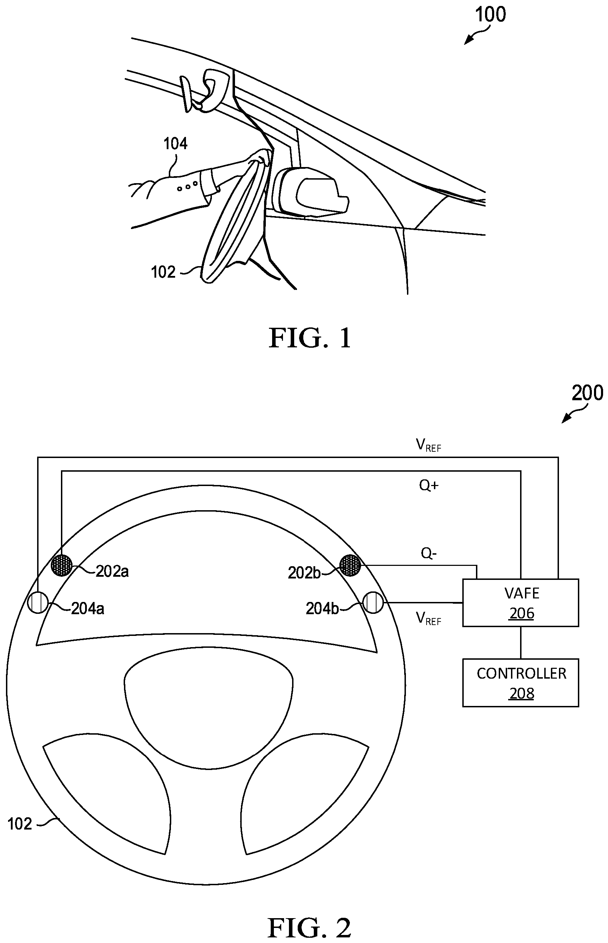

Figure 1 illustrates a vehicle interior environment 100 where a driver's hand 104 is positioned on a steering wheel 102, according to one or more embodiments disclosed herein. This configuration represents the context for hands-on detection (HoD) systems that monitor whether the driver's hands are properly placed on the steering wheel. Proper hand placement serves as a safety element in modern vehicles, particularly those equipped with advanced driver assistance systems (ADAS).

In the context of autonomous driving technologies, reliable hands-on detection has become increasingly valuable for ensuring driver readiness to intervene when necessary. For example, United Nations Regulation 79, adopted by the European Union for new production vehicles in April 2021, includes hands-on detection in all new cars with Lane Keeping Assist Systems (LKAS). This regulatory requirement relates to accurate and reliable detection methods verifying driver engagement while automated systems are active.

Several conventional approaches have been employed for hands-on detection, each with distinct limitations. For example, a torque-sensing method relies on measuring the torsion on a bar via strain gauges to determine the applied force, detecting the minute deflections produced when a driver grips the wheel. However, this approach can be easily fooled when a driver presses against the wheel with their leg instead of using their hands. As another example, capacitive sensing monitors changes in capacitance when the driver's hands come in contact with the wheel. Still, it fails when the driver wears gloves and can generate false detection signals due to moisture or humidity. Some systems have attempted to improve reliability through enhanced capacitive sensing based on in-phase and quadrature (I/Q) demodulation, which measures resistive and capacitive impedance elements, allowing operation under challenging conditions, including moisture presence or when the driver is wearing gloves.

Further, none of these techniques can distinguish whether the detected hands belong to the same individual, making it impossible to verify that a single driver is properly engaged with the steering wheel and preventing the detection of a valid ECG signal, which can only be obtained when both hands of the same person are in contact with the electrodes.

While hands-on detection primarily serves safety verification purposes, the same steering wheel interface shown in Figure 1 can also serve as a platform for driver health assistance through ECG monitoring. This represents an expanding area of automotive safety technology focused on monitoring drivers' physical and mental state while operating vehicles. Driver health assistance enhances road safety by supporting driver alertness and focus during vehicle operation. Despite promising developments, ECG monitoring from steering wheels is not yet a standard feature in commercial vehicles. However, several car manufacturers have announced plans to incorporate such systems in the future.

This technology encompasses sensors that detect physiological signals such as heart rate, breathing patterns, and other vital signs. Additionally, these systems can incorporate cameras to monitor facial expressions and eye movements, providing a comprehensive assessment of the driver's state.

The collected data undergoes algorithmic analysis to identify potential signs of fatigue, distraction, or other conditions that might impair driving ability. Based on this analysis, the system can issue alerts to the driver or, in certain circumstances, initiate vehicle control interventions to prevent accidents.

Despite the potential benefits of these health monitoring systems, implementing ECG monitoring specifically in steering wheels has presented numerous challenges in practice. For example, motion artifacts from movement and vibration can interfere with signal accuracy. Electrode placement can be difficult, especially with gloved hands or in the presence of sweat. Interference from other electrical devices in the vehicle can degrade signal quality. Driver comfort may be affected by sensor placement, potentially reducing compliance. Additional challenges include complex calibration procedures, ECG timing synchronization with steering wheel motion events, and computational demands on the vehicle's systems.

Previous approaches to ECG monitoring in steering wheels include capacitive ECG, optical ECG, conductive ECG, and inductive ECG. Capacitive ECG uses capacitive sensors embedded in the steering wheel to measure changes in the driver's ECG signal as they grip the wheel by detecting changes in the electrical field around the driver's hands. A drawback of capacitive ECG is that the system may experience electrode placement issues, interference, and motion artifacts. PPG uses photoplethysmography sensors to measure changes in blood volume in the driver's fingers by detecting changes in blood flow with light, but can struggle with calibration, ambient light interference, finger placement challenges (especially with gloved hands), and motion artifacts. Conductive ECG uses conductive materials embedded in the steering wheel to detect changes in the electrical field around the driver's hands for measuring heart rate and rhythm, but faces electrode placement challenges, interference, and motion artifacts. Inductive ECG employs coils embedded in the steering wheel to detect changes in the magnetic field around the driver's hands but can be affected by coil placement issues, interference, calibration requirements, and motion artifacts.

The configuration shown in Figure 1 represents the context for addressing these challenges through improved hands-on detection and health monitoring approaches. By integrating appropriate sensing technologies within the steering wheel structure, such as strategically positioned electrodes and advanced signal processing capabilities, the system can potentially overcome the limitations of previous methods while providing reliable driver engagement monitoring and health assistance capabilities
Figure 2 illustrates a hands-on detection (HoD) system 200, according to an embodiment of the disclosure. The system includes a steering wheel with electrodes to detect proper hand placement and monitor physiological signals. The steering wheel includes receiving electrodes 202a and 202b positioned at approximately the 10 o'clock and 2 o'clock positions, respectively, which are common hand placement locations for drivers. These electrodes serve as receiving electrodes for detecting signals. Adjacent to these main electrodes, on the backside of the steering wheel, are stimulus electrodes 204a and 204b.

The stimulus electrodes 204a and 204b provide a reference voltage that creates a stable working point for signal detection. This arrangement enables local polarization, establishing a constant bias for the signal that helps filter out environmental electrical noise near the driver's hands. By positioning these stimulus electrodes near the receiving electrodes, the system establishes a more stable working point for biopotential measurements, improving the signal-to-noise ratio for ECG detection. This local polarization technique can be particularly valuable in the electrically noisy environment of a vehicle cabin, where various electronic systems can generate interference.

The HoD system 200 includes a vertical analog front end (VAFE) circuit 206 coupled to the electrodes. The VAFE circuit 206 serves dual functions: it generates and provides a reference voltage to the stimulus electrodes 204a and 204b for transmission, and it processes the signals detected by the receiving electrodes 202a and 202b. The VAFE circuit 206 can also include motion-sensing capabilities to correlate physiological measurements with driving actions. A controller 208 is coupled to the VAFE circuit 206. It executes algorithms that analyze the electrodes' voltage signals and ECG signal morphology to verify proper hand placement and extract various physiological parameters. These parameters can include heart rate information, cardiac metrics such as heart rate variability, ECG waveform characteristics, and potential indicators of driver fatigue, stress, or other physiological states that might affect driving performance, as further detailed.

In embodiments, VAFE circuit 206 is a high-sensitivity measurement circuit that can detect small voltages and electrostatic field variations depending on the contact scenario. When hands are in direct contact with the electrodes, the VAFE circuit 206 primarily measures a small voltage difference (in the range of tens of microvolts) between the receiving electrodes 202a and 202b. In contactless scenarios, the same circuit functions as an electrostatic field variation sensor that detects changes in the electric field around the receiving electrodes.

Internally, the VAFE circuit 206 can include various electronic components such as high-impedance amplifiers (with input impedance of, for example, 10 gigaohms), analog-to-digital converters, and signal processing circuits designed for the sensitive measurements. The VAFE circuit 206 includes differential inputs that receive signals Q+ and Q-, which are coupled to the receiving electrodes 202a and 202b, respectively. This differential arrangement allows the VAFE circuit 206 to measure the potential difference between the two inputs while rejecting common-mode noise that might affect both inputs equally. The high input impedance of the VAFE circuit makes it versatile for direct contact biopotential measurements and contactless electric field sensing applications.

In embodiments, when measuring electrostatic charge variations, the VAFE circuit 206 operates in a passive mode through its high impedance stage without requiring an internal source of electric charge variation. The measurements are not based on single data points but rather on multiple samples taken at a specific sampling rate (e.g., in the range of 200 to 800 hertz, with some implementations extending to 3.2 kilohertz). This sampling approach allows the VAFE to determine electrostatic charge variations based on multiple measurements, improving reliability in the noisy environment of a vehicle cabin. Additionally, utilizing such high sampling rates provides greater time resolution of the acquired signals, which enables more effective filtering techniques and permits the acquisition of ECG signals with improved definition. This enhanced signal definition can be particularly advantageous for detecting subtle features in the ECG waveform that can be relevant for advanced cardiac analysis and more precise physiological monitoring.

For transmitting the reference voltage pattern, the VAFE circuit 206 can generate a specific digital sequence encoded as variations in the reference voltage (V_{REF}) that is output to the stimulus electrodes 204a and 204b. This pattern serves as a "key signal" that can be detected when a conductive medium, such as a human hand, bridges the stimulus and receiving electrodes. The VAFE circuit 206 transmits this pattern repeatedly at regular intervals (for example, every 300 to 400 milliseconds) with pause periods between transmissions to minimize power consumption and potential interference.

In embodiments of the disclosure, the stimulus electrodes 204a and 204b serve a dual function in the hands-on detection system. These electrodes can operate in two distinct modes controlled by the controller 208, which switches between these modes depending on the current phase of operation.

For example, during the contact check phase, the controller 208 configures the stimulus electrodes 204a and 204b to function as code transmitters. In this mode, the electrodes are biased with a varying binary code voltage pattern generated by the VAFE circuit 206. This voltage pattern creates a specific, recognizable signature that can be detected by the receiving electrodes 202a and 202b when a hand bridges the stimulus and receiving electrodes. The controller 208 analyzes this received pattern to verify physical contact with the steering wheel.

Conversely, during the ECG measurement phase, the controller 208 reconfigures the stimulus electrodes 204a and 204b to function as reference voltage providers. In this mode, the electrodes are biased with a clean, constant reference voltage (VREF) that establishes a stable working point for biopotential measurements. This local polarization technique creates a constant bias that helps filter out environmental electrical noise near the driver's hands, improving the signal-to-noise ratio for ECG detection.

The controller 208 can manage the timing of these mode switches, ensuring that the system alternates appropriately between contact checking and ECG measurement to maintain continuous verification of proper hand placement while optimizing signal quality for each function. This dual-mode operation maximizes the functionality of the electrodes while minimizing the total number of components required in the system.

The VAFE circuit 206 converts the analog electrostatic charge variation signals into digital data through analog-to-digital converters. Controller 208 then processes this digital data to decode and validate the received patterns and analyze the ECG signals. Integrating these functions into a single circuit allows for efficient signal processing and reduces susceptibility to external interference.

In embodiments, the VAFE circuit 206 and controller 208 can be placed within the steering wheel, creating a self-contained, hands-on detection system. This integration can reduce signal path lengths between the electrodes and processing components, improving signal quality and reducing susceptibility to external interference. However, the placement of these components is not limited to the steering wheel. In alternative embodiments, the VAFE circuit 206 and controller 208 is located elsewhere in the vehicle, such as in the steering column, dashboard, or a central processing unit, with appropriate connections to the electrodes in the steering wheel. This flexibility in component placement allows the system to be adapted to various vehicle designs and integration requirements while maintaining functionality.

In embodiments, the VAFE circuit 206 and controller 208 communication is implemented using standard communication protocols such as Inter-Integrated Circuit (I2C) or Serial Peripheral Interface (SPI). However, these examples are non-limiting, and other communication protocols or interfaces can be used depending on specific implementation requirements, system architecture, and performance considerations.

In embodiments, HoD system 200 performs a two-step verification process. First, it detects the presence of hands through coded voltage pattern transmissions between electrodes. The VAFE circuit 206 generates a specific binary voltage pattern and transmits it through the stimulus electrodes 204a and 204b. When a hand bridges the stimulus and receiving electrodes, the transmitted voltage pattern can be detected by the receiving electrodes 202a and 202b and processed by the VAFE circuit 206, providing initial verification of physical contact. This approach is distinct from the constant reference voltage used for biasing and noise shielding during ECG measurements. Second, once contact has been validated through the transmitted code, the system stops the code transmission to prevent interference and switches to analyzing ECG signals. These ECG signals that can be properly detected when both hands of the same person are correctly positioned on the wheel, offering a more definitive confirmation of proper driver engagement. The sequential operation-first validating contact through code transmission and then switching to ECG analysis with the transmission stopped-ensures optimal signal quality for each verification step.

The HoD system 200 incorporates multiple techniques for signal conditioning and noise reduction. The local polarization technique provided by the reference electrodes helps establish stable reference points for the left and right hands, making the signal less sensitive to environmental noise. For robust DC offset cancellation, the system can employ a combination of approaches, such as hardware-based high-pass filters with capacitors in series with the electrodes, digital filtering implemented within the VAFE circuit, and firmware-based filtering algorithms executed by the controller. This comprehensive approach to signal conditioning enables more accurate ECG measurements by effectively removing DC offset components while preserving the essential ECG signal characteristics.

Controller 208 can implement in-line ECG signal morphology checking algorithms that analyze the detected cardiac signals to verify their authenticity and filter out motion artifacts. For example, morphology checking can compare the detected signal patterns against expected ECG waveform characteristics, examining parameters such as signal amplitude, peak width, and beat-to-beat intervals.

By continuously analyzing these parameters, the system can distinguish between valid ECG signals and artifacts caused by steering wheel vibrations, hand movements, or other environmental factors. Advantageously, the proposed signal verification ensures that the system can reliably detect proper hand placement even in challenging driving conditions.

The electrode arrangement shown in Figure 2 represents one example configuration, but the disclosure contemplates various other arrangements with different numbers and positions of electrodes. The stimulus and receiving electrodes are not necessarily arranged, as shown in the figure. For example, depending on the specific implementation requirements, they can be positioned on the sides of the steering wheel, on the back surface, or in any combination of these locations. This flexibility in electrode placement allows the system to be adapted to different steering wheel designs and driver grip preferences.

In some embodiments, electrodes are distributed around the entire 360-degree circumference of the steering wheel, allowing for the detection of hand placement regardless of where the driver grips the wheel. Such comprehensive coverage can also enable the system to determine the specific location of hands on the steering wheel, which is useful for more sophisticated driver monitoring applications.

The spacing and number of electrodes can be determined based on specific application requirements. Implementations with greater numbers of electrodes can provide better coverage and more precise hand position detection but can come at the cost of increased data processing requirements. System designers can select an appropriate compromise between coverage and complexity based on application needs, vehicle type, and desired functionality. Accordingly, the electrode configuration is not limited to what is shown in Figure 2, as various patterns and densities are employed to achieve different performance characteristics.

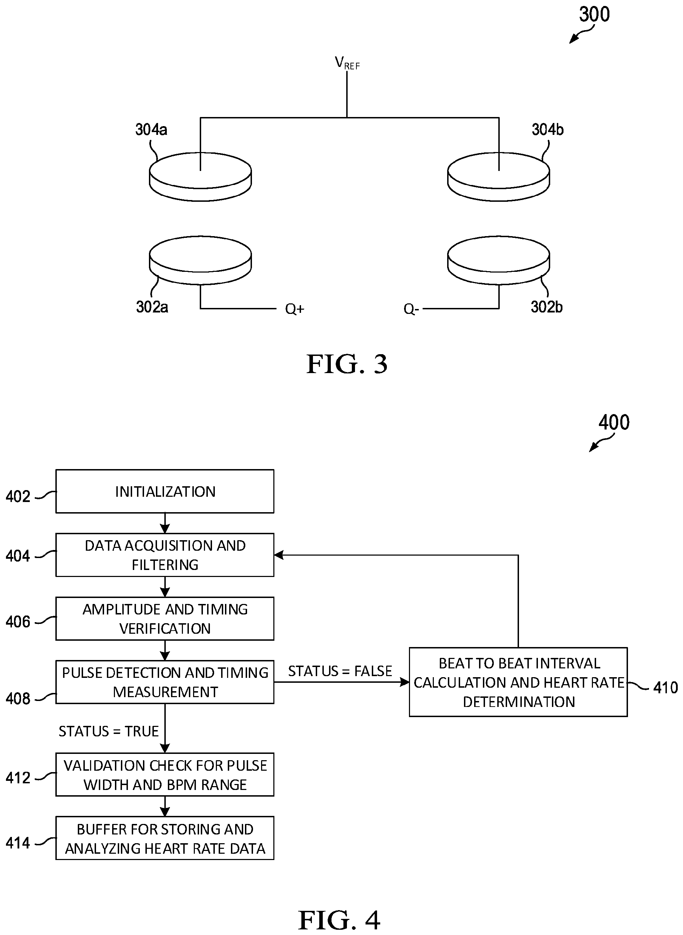

Figure 3 illustrates an electrode configuration 300 for hands-on detection, according to an embodiment of the disclosure. The configuration shows the electrical connections between the electrodes and the vertical analog front end (VAFE) circuit previously introduced in Figure 2. As a non-limiting example, the system includes four electrode pads arranged in a pattern representing their positioning on a steering wheel, expanding on the conceptual arrangement shown in Figure 2.

The stimulus electrodes 304a and 304b are coupled to a reference voltage (V_{REF}). These electrodes are constructed from conductive materials, such as copper, aluminum, or conductive polymers, to transmit electrical signals efficiently. The electrodes can have a circular disc shape, as shown in the figure. Still, other shapes, such as rectangular, oval, or contoured geometries, may be used to conform to the steering wheel's surface and optimize contact with the driver's hands. These electrodes' surface area and thickness can be tailored to balance signal transmission efficiency with manufacturing and integration considerations. In some embodiments, the electrodes are coated with a thin insulating layer to prevent direct electrical contact while allowing capacitive coupling.

In embodiments, the stimulus electrodes transmit a reference voltage pattern as a stimulus signal. This arrangement corresponds to the stimulus electrodes 204a and 204b shown in Figure 2 but with more detail regarding their electrical coupling. In operation, the reference voltage pattern comprises a specific digital sequence that can be encoded as variations in the reference voltage (V_{REF}). This pattern functions as a "key signal" that can be detected and validated by the receiving system.

The signal code transmission for hands positioning utilizes an encoding technique to create a distinctive reference voltage pattern. This pattern can be implemented as a digital word with a predetermined sequence of bits, where a voltage level transition represents each bit. For example, a binary "1" might be encoded as a high-to-low transition, while a binary "0" might be encoded as a low-to-high transition. The complete digital word forms a unique signature that can be reliably detected and distinguished from random environmental noise or interference.

In some embodiments, the digital word includes redundancy features such as error correction codes or checksums to enhance detection reliability. The system is also configured to employ techniques such as interleaving zeros between key bits to create a more distinctive pattern that is less likely to occur naturally in the environment. For example, a simple 8-bit code might be expanded to a 16-bit transmission by inserting zeros between each original bit. The zero-interleaving technique forces transitions between "1" and "0" states in the transmitted signal, which simplifies the decoding process of continuous sequences of "1" bits that might otherwise be difficult to distinguish. These forced transitions create clear voltage level changes that are more readily detectable by the receiving circuitry, improving the overall robustness of the code transmission and reception process.

The voltage pattern can follow carefully timed sequences. For example, each bit in code requires approximately 15 to 25 milliseconds to transit, with a complete code transmission take 120-400 milliseconds to be completed, depending on the code length. After transmitting the complete pattern, the system pauses transmission for a predetermined period, such as 100 to 200 milliseconds, before repeating the cycle. The pause duration can be adaptive based on, for example, vehicle conditions. For example, if the embedded accelerometer detects no vibration over a programmable threshold, suggesting the vehicle might not be moving, the pattern repetition frequency can be reduced over time, extending the pause to, for example, 0.5 to 1 second. The adaptive timing structure allows the system to continuously monitor for hand presence while optimizing power consumption and minimizing potential interference with other vehicle systems based on the current driving context. It should be appreciated that the timing parameters discussed herein are to aid the discussion and not necessarily limiting, as these values can be adaptable or programmable based on various parameters including vehicle type, driving conditions, user preferences, power management requirements, and system integration considerations.

The stimulus electrodes 304a-b transmits the reference voltage pattern simultaneously or in a coordinated sequence. In some embodiments, each stimulus electrode transmits a different portion of the pattern, requiring the presence of both hands to complete the full pattern detection. This approach can ensure that the driver has both hands properly positioned on the steering wheel rather than just one hand covering multiple electrodes.

An example implementation of the signal code transmission type of encoding technique, albeit in a different application, is provided in commonly assigned U.S. Patent No. 11,989,065, which is incorporated herein by reference in its entirety.

The receiving electrodes 302a-b are coupled to the Q+ and Q- differential inputs of the VAFE circuit 206. Like the stimulus electrodes, these receiving electrodes are made from conductive materials to maximize signal reception. The material selection can prioritize conductivity, durability, and resistance to oxidation to ensure long-term reliability in the automotive environment. These electrodes correspond to the receiving electrodes 202a and 202b shown in Figure 2. The shape and size of the receiving electrodes 302a-b can be optimized for maximum sensitivity to electrostatic charge variations, potentially differing from the stimulus electrodes 304a-b to serve their specific function more effectively.

When a driver's hands bridge the stimulus and receiving electrodes, electrostatic charge variation occurs between the electrodes. Human skin, with its moderate conductivity due to moisture and salt content, serves as a conductive medium that completes the circuit between the stimulus and receiving electrodes. This conductivity property of human skin allows for the system's operation and helps distinguish between human hands and non-conductive objects that might touch the steering wheel.

The signal transmission between electrodes can work through a combination of coupling mechanisms. For example, when no hands are present, the stimulus and receiving electrodes have minimal coupling. However, when a hand is placed on the steering wheel covering the stimulus and receiving electrodes, the hands act as a conductive medium that enhances the signal transmission. This coupling can occur through, for example, capacitive and resistive mechanisms. When the hand is in direct contact with the electrodes (without gloves), resistive coupling predominates as the skin provides a direct conductive path. When the hand is separated from the electrodes by thin insulation or gloves, capacitive coupling becomes the primary mechanism. This versatility in coupling mechanisms allows the system to function in various conditions, including when the driver is wearing gloves. This change in coupling causes a measurable variation in the signal detected at the receiving electrodes 302a-b.

The electrode materials and construction must balance several factors: sufficient conductivity to function effectively, durability to withstand the automotive environment, and appropriate surface properties to ensure reliable contact with hands in various conditions (dry, moist, or gloved). In some embodiments, the electrodes are integrated directly into the steering wheel structure, potentially beneath a thin material layer that preserves the steering wheel's tactile feel while allowing electrostatic charge variation to be detected.

The VAFE circuit 206 measures this electrostatic charge variation through its Q+ and Q- inputs. The measurement process involves detecting changes in the electrical field around the receiving electrodes 302a and 302b. The VAFE circuit 206 includes detects small variations in electrostatic charges, such as high-impedance amplifiers and analog-to-digital converters. These components convert the analog electrostatic charge variation signals into digital data that the controller can process.

In embodiments, the voltage pattern transmitted through the stimulus electrodes 304a-b is designed to create a specific, recognizable pattern of electrostatic charge variations at the receiving electrodes. This pattern serves as the key signal the system looks for to confirm hand presence. Controller 208 can process the digitized electrostatic charge variation data to decode and validate the received pattern against the expected key signal pattern.

The validation process by controller 208 can involve steps to ensure accurate detection. In embodiments, controller 208 analyzes the received electrostatic charge variation data to extract the pattern. This extraction involves sampling the received signal at specific intervals corresponding to the expected bit transitions in the transmitted pattern. Controller 208 can then apply filtering algorithms to remove noise and enhance the signal quality. Next, it decodes the filtered signal to recover the bit pattern, which can be compared with the expected key signal pattern. The comparison uses correlation techniques or direct bit-by-bit matching with allowance for a certain number of bit errors. If the patterns match within acceptable tolerance limits, the system confirms that a hand is bridging the stimulus and receiving electrodes.

In embodiments, the system requires multiple consecutive successful detections before confirming hand presence to prevent false positives. Similarly, the system maintains a positive detection status to avoid false negatives from momentary signal disruptions unless multiple consecutive detection failures occur. This process is similar to how a key fits a lock-only the correct pattern (key) will result in a positive validation.

The differential arrangement of the Q+ and Q- inputs helps reject common-mode noise that might affect both inputs equally. This configuration enhances the system's ability to detect the reference voltage pattern reliably, even in the electrically noisy environment of a vehicle cabin. The controller can also apply additional filtering and signal processing techniques to improve the signal-to-noise ratio of the received electrostatic charge variation data.

In the context of hands-on detection for steering wheels, this electrode configuration enables the first verification step of the two-step process described in Figure 2. The system first confirms physical contact through the detection and validation of the reference voltage pattern via electrostatic charge variation. Once this initial verification is complete, the same electrodes can be used for the second verification step involving ECG signal analysis.

In embodiments, the electrodes that detect electrostatic charge variations can capture the driver's electrocardiogram signals when both hands are in contact with the steering wheel. In this ECG measurement mode, the receiving electrodes 302a-b function as the primary ECG detection points, while the reference electrodes 304a-b provide local polarization to enhance signal quality.

For ECG measurement, the local polarization technique can be particularly advantageous. The reference voltage (V_{REF}) applied to electrodes 304a-b creates stable electrical reference points near each hand position. This local polarization establishes a constant bias that helps filter out environmental electrical noise near the hands. The proximity of these reference electrodes to the measurement points creates a more stable working point for the small biopotential measurements involved in ECG detection. For robust DC offset cancellation, the system can employ a combination of approaches, such as hardware-based high-pass filters with capacitors in series with the electrodes, digital filtering implemented within the VAFE circuit, and firmware-based filtering algorithms executed by a controller. This comprehensive approach to signal conditioning enables more accurate ECG measurements by effectively removing DC offset components while preserving the ECG signal characteristics, particularly important in an electrically noisy vehicle environment. The proximity of these reference electrodes to the measurement points is typically within 2 to 5 millimeters, creating a more stable working point for the small biopotential measurements involved in ECG detection.

When a driver places both hands on the steering wheel, each hand contacting a pair of electrodes (one stimulus and one receiving electrode), an electrical path is created between the left and right hands through the driver's torso. This path allows the system to detect the small voltage differences generated by cardiac activity. The electrical potential difference between the two hands typically falls within the detectable millivolt range, enabling the VAFE circuit 206 to capture the ECG signal through its Q+ and Q- differential inputs.

Advantageously, the differential measurement between electrodes 302a-b allows for accurate ECG detection. The Q+ input receives signals from receiving electrode 302a (e.g., in contact with the left hand), while the Q- input receives signals from receiving electrode 302b (e.g., in contact with the right hand). This arrangement allows the VAFE circuit 206 to measure the potential difference between the two hands, which contains the ECG signal information. The differential measurement also helps reject common-mode noise, which can affect both inputs equally.

Once the ECG signal is captured, controller 208 can perform a morphology check to verify that the detected signal represents a genuine cardiac signal rather than noise or artifacts. This morphology check analyzes the waveform characteristics of the detected signal, examining features such as the presence of waves associated with the electrical depolarization of the atria (P waves), the electrical depolarization of the ventricles (QRS complexes), and ventricular repolarization (T waves) with appropriate amplitude relationships and timing intervals. Controller 208 can also analyze beat-to-beat intervals and heart rate variability to confirm signal validity.

The morphology check can be particularly advantageous for ensuring robustness against motion artifacts. During normal driving, the steering wheel may experience vibrations, and the driver's hands may shift slightly, potentially introducing artifacts into the ECG signal. By analyzing the morphological characteristics of the detected signal, the system can distinguish between genuine cardiac signals and motion-induced artifacts, ensuring reliable hands-on detection even in challenging driving conditions.

The combination of local polarization for signal quality enhancement and morphology checking for signal validation enables the system to reliably detect and confirm that both hands of the same person are properly positioned on the steering wheel. This dual verification approach-first through electrostatic charge variation detection and then through ECG signal analysis-provides a highly reliable method for hands-on detection resistant to potential circumvention attempts.

Figure 4 illustrates a flowchart of method 400 for electrocardiogram (ECG) signal processing and morphology checking, according to an embodiment of the disclosure. This method verifies proper hand placement on a steering wheel by analyzing ECG signals detected between the driver's hands. Method 400 extracts valid heart rate information while rejecting noise and motion artifacts. In some embodiments, the method includes additional or fewer steps, or the steps can be performed differently than illustrated.

At step 402, system variables are set to their starting values. For example, the initialization step can include setting the time between detected pulses (d_{T}) and the start time (T₀) to zero, beats per minute (BPM) to zero, pulse detection flag to FALSE, recording the current time as the pulse start time (Pulse T_{ON}), setting the status flag to FALSE, and initializing pulse width, pulse frequency, and standard deviation of BPM values to zero. The initialization step ensures that all variables start from a known state before signal processing begins.

At step 404, following initialization, the system continuously acquires sensor data from the electrodes positioned on the steering wheel. In embodiments, the acquired data undergoes filtering operations to improve signal quality. For example, these operations can include one or of applying a moving average filter to smooth the signal, implementing a notch filter to attenuate powerline noise, and processing the signal through an infinite impulse response (IIR) high-pass filter with a cutoff frequency of approximately 1 Hz to remove baseline drift. Advantageously, the filtering steps allow for extracting the ECG signal from the noisy environment of a moving vehicle.

At step 406, a check is performed to determine whether the amplitude of the filtered signal falls within a predetermined acceptable range defined by minimum (A_{MIN}) and maximum (A_{MAX}) threshold values. If the amplitude is outside this range, the signal may be too weak to represent a valid ECG or may contain excessive noise.

At step 408, the system performs a pulse detection and timing measurement. In response to a successful amplitude verification, corresponding to the amplitude of the filtered signal being within the predetermined acceptable range, the system sets a pulse detection flag to TRUE. It records the start time (T₀) of the pulse to the current time. If the time between detected pulses (d_{T}) is less than a minimum threshold (d_{T_MIN}), the system generates a first error flag suggesting that the signal is noisy. The time between detected pulses (d_{T}) corresponds to the time between the current and detected pulse immediately before the current pulse. In an embodiment, the minimum threshold (d_{T_MIN}) is approximately 375 milliseconds, corresponding to a BPM of about 160.

In contrast, if the amplitude of the filtered signal is outside the predetermined acceptable range, the system sets the pulse detection flag to FALSE. It also sets the time between detected pulses (dₜ) to the current time subtracted by the start time (T₀).

Regardless of the amplitude verification status, if the time between detected pulses (d_{T}) is greater than a maximum threshold (d_{T_MAX}), the system generates a second error flag suggesting no signal has been detected. This check allows the system to flag instances where too much time has passed without detecting a pulse. In an embodiment, the maximum threshold (d_{T_MAX}) is approximately 1500 milliseconds, corresponding to a BPM of about 40.

If any error flags are generated at step 408, the system transitions back to step 404 to collect more data. However, in response to no error flags being set, the system determines whether the status flag is set to TRUE or FALSE.

At step 410, if the status flag is set to FALSE, the system performs a beat-to-beat interval calculation and heart rate determination. If the pulse detection flag is set to TRUE, the system calculates the time between detected pulses (d_{T}). The pulse frequency can be computed as the inverse of time between detected pulses (d_{T}) (i.e., pulse frequency equal to 1/d_{T}). The heart rate in beats per minute (BPM) can be calculated using one of several standardized procedures or as an optimized value based on multiple procedures. In the implemented algorithm, the system can adopt a straightforward approach: measuring the average peak interval over a six-second window, taking the inverse to convert from time to frequency, and then estimating the BPM by extrapolating this value over 60 seconds (multiplying by 60). This method balances responsiveness to changes in heart rate and stability of measurement. The system also records the pulse start time (Pulse T_{ON}) to the current time for the next cycle. In embodiments, this information can be stored in a first register, such as in a first-in-first-out (FIFO) buffer or a memory storage. In addition, the standard deviation of the BPM values is calculated as the standard deviation of the values stored in the first register.

Once the beat-to-beat interval calculation and heart rate determination are completed, the method transitions back to step 404 to collect more data. If the pulse detection flag is set to FALSE, the system transitions immediately to step 404 without performing any computational steps.

At step 412, if the status flag is set to TRUE after step 408, the method performs a validation check for pulse width and BPM range. The records the current time as the pulse stop time (Pulse T_{OFF}). In addition, the system sets the status flag to FALSE.

In response to the pulse detection flag being FALSE, the system records the current time as the pulse stop time (Pulse T_{OFF}). The pulse width is then calculated by subtracting the pulse start time (Pulse T_{ON}) from the pulse stop time (Pulse T_{OFF}). If the pulse detection flag is set to TRUE, the system transitions immediately to step 404 without performing any computational steps.

The system verifies whether the pulse width falls within an acceptable range defined by minimum (PW_{MIN}) and maximum (PW_{MAX}) threshold values. If the pulse width is outside this range, the system generates an error flag indicating that the peak width is out of the normal range. In an embodiment, the minimum (PW_{MIN}) threshold value is 2 milliseconds. In an embodiment, the maximum (PW_{MAX}) threshold value is 20 milliseconds.

The system checks whether the current BPM value stored in the first register fall within an acceptable physiological range (typically 40-180 BPM for adults). If the value is outside this range, the system generates an error flag indicating that the BPM value is out of the normal range.

Next, the system checks whether the standard deviation of the BPM values calculated at step 410 and stored in the first register is less than a maximum threshold (SD_{BPM_MAX}). This check ensures that the heart rate measurements are consistent and stable over time. If the standard deviation exceeds the maximum threshold (SD_{BPM_MAX}), the system generates an error flag indicating that the BPM value is too different from previous measurements. This check is particularly valuable for detecting irregular heartbeats or motion artifacts that might cause the heart rate measurements to fluctuate excessively. In an embodiment, the maximum threshold (SD_{BPM_MAX}) is in the order of 2 to 5.

Table 1 illustrates standard ECG parameters that the system can use during the morphology check process. These parameters represent the typical characteristics of a normal ECG waveform, including the durations and amplitudes of various components. During the validation process, the system can compare measured values against these standard ranges to verify that the detected signal represents a genuine cardiac signal rather than noise or artifacts.

**Table I: ECG Parameters**

| **Section** | **Durations (ms)** | **Amplitude (mV)** | **Definition** |
|---|---|---|---|
| P Waves | 70 to 120 | 0.2 to 0.4 | Atrial Depolarization |
| QRS Complex | 60 to 100 | 1 to 2 | Ventricular Depolarization |
| PR Interval | 120 to 200 | - | Atrioventricular Conduction Time |
| T Waves | 180 to 200 | 0.4 to 0.5 | Ventricular Repolarization |
| ST Interval | 300 | - | Duration of Ventricular Repolarization |
| QT Interval | 400 | - | Duration of Ventricular Electrical Systole |
| RR Interval | 800 to 900 | - | Duration of the Cardiac Cycle |

As shown in Table 1, P waves, representing atrial depolarization, typically have a duration of 70 to 120 milliseconds and an amplitude of 0.2 to 0.4 millivolts. The QRS complex, representing ventricular depolarization, has a 60 to 100 milliseconds duration and a larger amplitude of 1 to 2 millivolts, making it the most prominent feature of the ECG waveform. The PR interval, which measures the atrioventricular conduction time, ranges from 120 to 200 milliseconds. T waves, representing ventricular repolarization, have a duration of 180 to 200 milliseconds and an amplitude of 0.4 to 0.5 millivolts. The ST interval, measuring the duration of ventricular repolarization, is approximately 300 milliseconds, while the QT interval, representing the duration of ventricular electrical systole, is approximately 400 milliseconds. The RR interval, which corresponds to the duration of the complete cardiac cycle, typically ranges from 800 to 900 milliseconds.

In embodiments, the system can use these parameters during the morphology check to verify that the detected signal exhibits the expected characteristics of a genuine ECG. For example, the system verifies that the detected QRS complex has an appropriate amplitude relative to other components, that intervals between successive waves fall within expected ranges, and that the overall waveform morphology matches the expected pattern. Deviations from these standard parameters indicate either signal artifacts or potential cardiac abnormalities. By analyzing these parameters, the system can distinguish between valid ECG signals and motion artifacts or other interference, ensuring reliable hands-on detection even in challenging driving conditions.

These parameters allow for evaluating not only the presence of hands on the steering wheel but potentially also the state of the heart's health. Variations from these standard parameters may indicate cardiac anomalies, pathologies, or arrhythmias, which could be used in advanced implementations to provide additional health monitoring capabilities beyond basic hands-on detection.

At step 414, valid BPM values that pass the previous validation checks are stored in a second register (e.g., a second FIFO buffer). In embodiments, the average BPM values stored in the second register are calculated and stored in the second register. This register allows the system to maintain a history of recent heart rate measurements for statistical analysis and trend detection. Using multiple buffers enables the system to perform different types of analysis on the heart rate data at different processing stages.

In embodiments, method 400 implements an overlapping window approach. This approach requires at least three peaks of the cardiac cycle to make a reliable determination of heart rate. The system analyzes overlapping data points (e.g., points 1-2-3, then 2-3-4, then 3-4-5, and so on) in a shift window manner. This approach allows the system to update its heart rate determination while maintaining historical context. The average BPM is calculated from the values stored in the second register to provide a stable heart rate measurement less susceptible to momentary variations.

The various error flags (or error codes) help the system determine whether a valid ECG signal is present, confirming whether both hands of the same person are properly positioned on the steering wheel.

In embodiments, method 400 operates continuously while the hands-on detection system is active, constantly updating its determination of whether valid hand contact is maintained. This continuous monitoring capability is advantageous for advanced driver assistance systems that require ongoing verification of driver engagement.

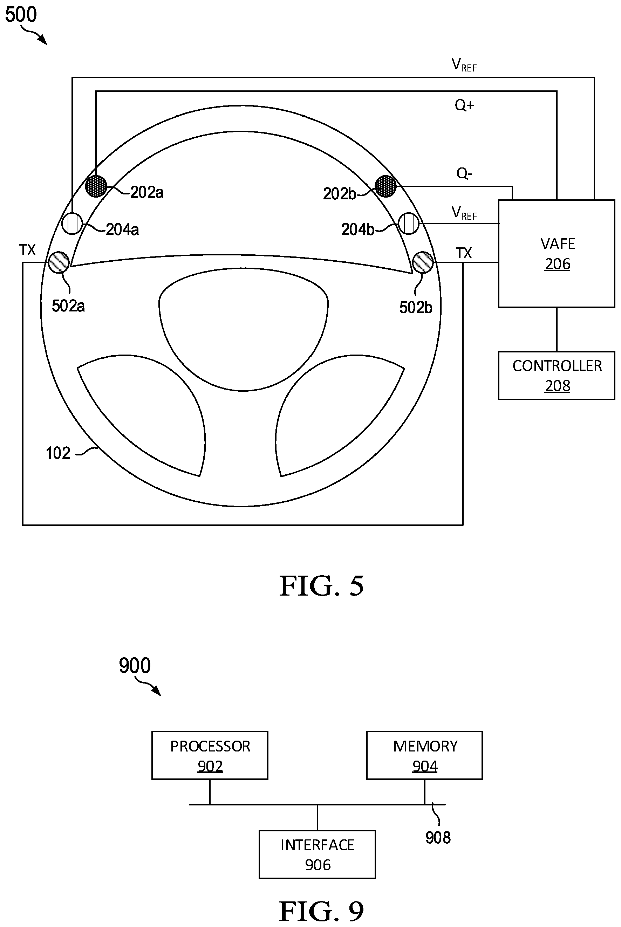

Figure 5 illustrates a hands-on detection (HoD) system 500 for operation without hands tracking, according to an embodiment of the disclosure. This configuration is designed to detect the presence of hands on the steering wheel without specifically tracking their exact positions around the wheel circumference. This embodiment expands upon the configuration shown in Figure 2, incorporating additional electrodes to enhance detection capabilities while maintaining similar principles of operation.

HoD system 500 includes a steering wheel with multiple electrodes to detect proper hand placement and monitor physiological signals. The receiving electrodes 202a and 202b are positioned at approximately the 10 o'clock and 2 o'clock positions, respectively, and are coupled to the Q+ and Q- differential inputs of the VAFE circuit 206. These electrodes serve as the primary contact points for ECG signal detection.

Adjacent to the main receiving electrodes 202a-b are local polarization reference electrodes 204a-b, which are coupled to the reference voltage (V_{REF}) output of the VAFE circuit 206. These reference electrodes 204a-b provide a stable working point for signal detection by establishing a constant bias that helps filter out environmental electrical noise near the driver's hands.

In addition to these electrodes, HoD system 500 includes dedicated transmitter electrodes 502a and 502b positioned below the reference electrodes. These transmitter electrodes are coupled to the output of the VAFE circuit 206. The transmitter electrodes 502a-b are configured to transmit the reference voltage pattern for the first verification step of the hands-on detection process.

In embodiments, the system verifies the placement of the driver's first hand by transmitting a code (reference voltage pattern) through transmitter electrode 502a and detecting it through receiving electrode 202a when the driver's hand bridges these electrodes. Similarly, the system verifies the second hand by transmitting a code through transmitter electrode 502b and detecting it through receiving electrode 202b.

If the first-hand and second-hand verifications are successful, the system executes the morphology check and heartbeat computation using the ECG signals detected between the receiving electrodes 202a-b. The local polarization provided by reference electrodes 204a-b creates a stable working point that filters out noise from the environment near the hands, enhancing the quality of the ECG signal.

If the ECG morphology check is verified, the system confirms that both hands are properly positioned on the steering wheel. This dual verification approach-through code transmission and ECG analysis-provides a reliable method for hands-on detection that is resistant to potential circumvention attempts.

The VAFE circuit 206 and controller 208 process the signals from all electrodes. In embodiments, the VAFE transmits the reference voltage patterns and the reception and processing of ECG signals. Controller 208 executes the algorithms that analyze the received signals and make the final determination regarding proper hand placement.

This configuration, with dedicated electrodes for each function (receiving, reference, and transmission), provides enhanced performance compared to configurations where electrodes serve multiple functions. Separating these functions allows for optimizing each electrode type for its specific purpose, potentially improving signal quality and detection reliability.

The configuration shown in Figure 5 differs from that in Figure 2 by introducing dedicated transmitter electrodes 502a-b. While the reference electrodes 204a-b in Figure 2 could serve dual purposes (providing local polarization and transmitting code signals), having dedicated electrodes for each function offers several technical advantages. When electrodes perform multiple functions, design compromises are often necessary. For example, an electrode optimized for providing a stable reference voltage might not be ideal for transmitting clear code signals, and vice versa.

The dedicated transmitter electrodes 502a-b allow for the optimization of code transmission without affecting the local polarization function. This separation can improve signal integrity in several ways. First, the transmitter electrodes can be designed with different materials, shapes, or surface areas that enhance transmission strength and clarity without compromising the reference voltage stability. Second, if combined, the transmitter electrodes can operate at different voltage levels or frequencies optimized for code transmission, which might interfere with the stable reference voltage. Third, having separate electrodes eliminates potential switching artifacts when alternating a single electrode between reference and transmission functions.

Additionally, separating these functions can simplify the control circuitry in the VAFE circuit 206, as it doesn't need to rapidly switch electrode functions. This can reduce circuit complexity and potential sources of noise or interference. The dedicated transmitter electrodes permit a simplification of the process by avoiding functionality switching on the electrodes. Indeed, using both kinds of electrodes (dedicated transmitters and reference electrodes), it is possible to continuously maintain the reference voltage biasing even during code transmission, providing improved noise shielding through all operational phases. The continuous noise shielding can be particularly advantageous in the electrically noisy environment of a vehicle cabin. While this approach requires additional electrodes and connections, the benefits in terms of signal quality, system performance, and reliability can justify this design choice, particularly for safety-critical applications like hands-on detection in advanced driver assistance systems.

HoD system 500 is specifically designed for hands-on detection without hands-tracking functionality. This means the system can reliably verify that both hands are properly positioned on the steering wheel but does not attempt to continuously track the specific locations of the hands as they move around the wheel during driving maneuvers. This focused approach allows for a simpler electrode configuration while providing the essential safety verification required for advanced driver assistance systems. For applications where hand tracking is desired, additional electrodes distributed around the steering wheel circumference are employed, as discussed concerning other embodiments of the disclosure.

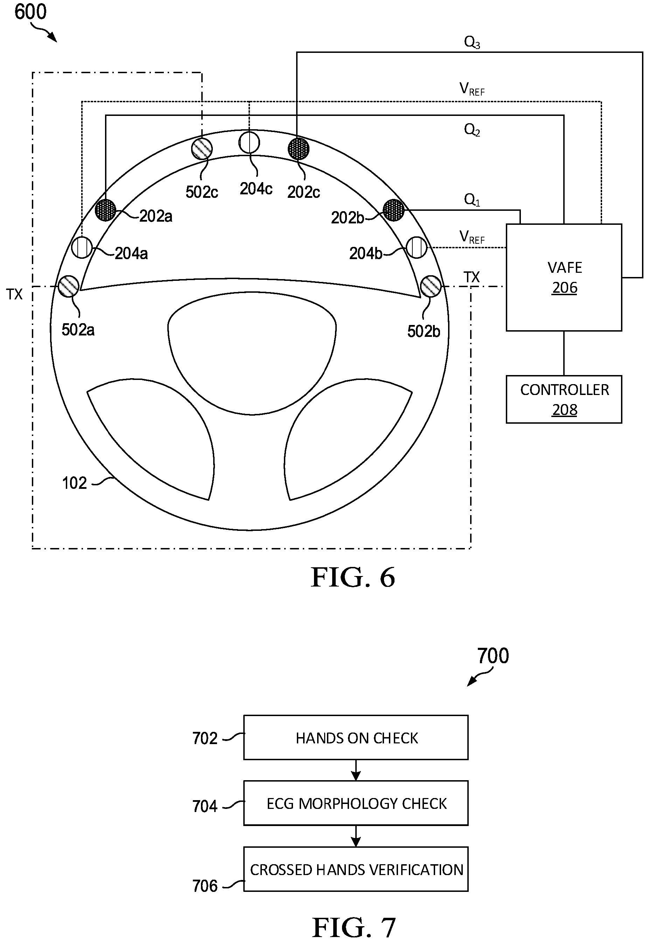

Figure 6 illustrates a hands-on detection (HoD) system 600 for operation with hands-tracking capability, according to an embodiment of the disclosure. This configuration expands upon the systems shown in Figures 2 and 5, incorporating additional electrodes and scanning functionality to detect the presence of hands on the steering wheel and track their positions during driving.

HoD system 600 includes multiple electrodes distributed around the steering wheel circumference. In addition to the electrode sets at the 10 o'clock and 2 o'clock positions (202a-b, 204a-b, 502a-b) similar to those shown in Figure 5, the system includes an additional set of electrodes (202c, 204c, 502c) positioned at the top of the steering wheel. This expanded electrode arrangement enables the system to detect hand placement at various positions around the wheel.

The receiving electrodes 202a-c are coupled to multiple inputs of the VAFE circuit 206, allowing for differential measurements between various electrode pairs. The reference electrodes 204a-c are coupled to the reference voltage (V_{REF}) output of the VAFE circuit 206, providing local polarization at multiple positions around the wheel. The transmitter electrodes 502a-c are coupled to the output of the VAFE circuit 206 for transmitting code signals at various positions.

It should be understood that the number, type, and arrangement of electrodes shown in Figure 6 are exemplary and non-limiting. The disclosure contemplates various other electrode configurations with different numbers, spacings, and arrangements of receiving, reference, and transmitter electrodes distributed around the steering wheel. The specific configuration is adapted based on the steering wheel design, desired detection resolution, and system performance requirements. For example, some embodiments include more or fewer electrode sets, different spacing between electrodes, or alternative placement patterns around the wheel circumference.

Figure 7 illustrates a method for operating the HoD system 600, according to an embodiment of the disclosure. In some embodiments, the method includes additional or fewer steps, or the steps can be performed differently than illustrated.

At step 702, HoD system 600 scans 1 to N electrodes, where N is an integer representing the number of electrode sets distributed around the steering wheel-in the example of HoD system 600, N equals three. This scanning process checks each electrode position to detect hand contact. The system transmits code signals through each transmitter electrode 502a-c in sequence and monitors the corresponding receiving electrodes 202a-c for detection.

At step 704, HoD system 600 performs an ECG morphology check for each position where hand contact is detected through the transmit and receive code communication between the electrodes. In embodiments, the check involves analyzing the ECG signals detected between the relevant electrode pairs to verify a valid cardiac signal. The system can perform this analysis on multiple detected positions, allowing it to determine exactly where the driver's hands are placed on the wheel.

At step 706, HoD system 600 detects crossed hands by analyzing the QRS complex inversion in the ECG signal. When a driver crosses their hands on the steering wheel (for example, during a turning maneuver), the polarity of the ECG signal becomes inverted compared to the normal hand position. By detecting this inversion, the system can determine that both hands are on the wheel and are in a crossed position.

In embodiments, the VAFE circuit 206 and controller 208 work together to process the signals from all electrodes. The VAFE circuit 206 can handle the transmission of code signals, the provision of reference voltages, and the reception and processing of ECG signals from multiple positions. Controller 208 executes the algorithms that manage the scanning process, analyze the received signals, and determine hand placement and position determinations.

The hands-tracking capability of HoD system 600 provides several advantages over the systems shown in Figures 2 and 5. By knowing the specific positions of the driver's hands, the system can interpret driver intent and potentially provide more nuanced feedback to advanced driver assistance systems. For example, it can distinguish between normal driving grip, turning maneuvers with crossed hands, and one-handed driving situations.

The scanning approach also enables the system to function regardless of where the driver chooses to grip the steering wheel, accommodating different driving styles and hand positions. This flexibility enhances the user experience while maintaining the safety benefits of reliable hands-on detection.

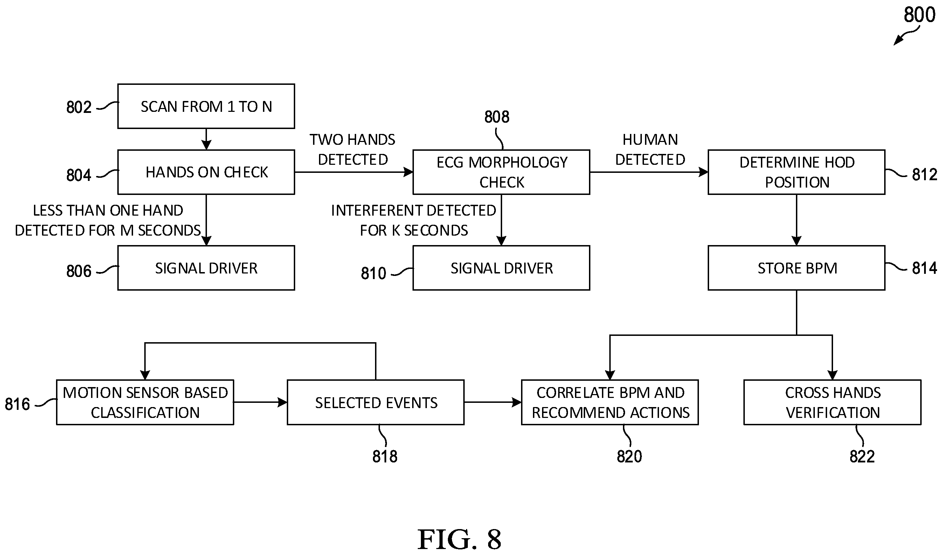

Figure 8 illustrates a flowchart of a method 800 for hands-on detection, heartbeat computation, and health assistance, according to an embodiment of the disclosure. This method integrates the hands-on detection capabilities with driver health monitoring and assistance features, providing a system for ensuring driver engagement and well-being. In embodiments, the method includes additional or fewer steps, or the steps can be performed differently than illustrated.

At step 802, the hands-on-detection system begins with a scanning operation from 1 to N, where N represents the number of electrode sets distributed around the steering wheel. This scanning process systematically checks each electrode position to detect hand contact, similar to the approach described concerning Figure 6.

At step 804, the system performs a hands-on check using the transmit-receive code transmission technique previously disclosed. If two hands are detected, the method proceeds to step 808 for ECG morphology checking. However, if less than one hand is detected for a predetermined period (M seconds), the method branches to step 806, where the system signals the driver. This signal includes a visual or audible alert requesting the driver to place both hands on the steering wheel. In some embodiments, certain vehicle functions are temporarily disabled until proper hand placement is detected.

At step 808, the system performs an ECG morphology check on the detected positions to verify that a valid cardiac signal is present, confirming that a human is in contact with the electrodes rather than an inanimate object. If an interferent (non-human object) is detected for a predetermined period (K seconds), the method branches to step 810, where the system signals the driver. This signal might include a message to remove gloves or other objects that might interfere with proper detection, and again, certain vehicle functions can be temporarily disabled.

If a human is detected through successful ECG morphology verification, the method proceeds to step 812, where the system determines the hands-on detection (HOD) position. This step identifies the specific locations where the driver's hands are contacting the steering wheel, enabling more precise monitoring of driver engagement.

At step 814, the system stores the beats per minute (BPM) data calculated from the ECG signal.

At step 816, the system performs motion sensor-based classification using data from the accelerometers and gyroscopes embedded in, for example, the same chip as the VAFE circuit. This classification identifies specific driving maneuvers or actions.

At step 818, the system identifies selected events from the motion sensor data, such as U-turns or other significant driving maneuvers that might affect the driver's physiological state. Other sensors, such as external cameras, radar systems, lidar systems, imaging systems, and the like, can be used in addition to motion sensor data.

At step 822, the system performs crossed-hands verification by analyzing the QRS complex inversion in the ECG signal, as described concerning Figure 6.

The motion sensor classification (step 816) and selected events (step 818) feed into step 820, where the system correlates the BPM data collected at step 814 with the selected events and recommends actions based on this analysis. For example, if the system detects an elevated heart rate during a challenging driving maneuver, it might recommend that the driver take a break or adjust their driving behavior.

The accelerometer can be used to determine the user's activity. For example, detecting that the steering wheel is rotating can avoid triggering unnecessary alerts when the driver is actively maneuvering the vehicle, which might temporarily require different hand positions.

This integrated approach combines hands-on detection, ECG monitoring, and motion sensing to provide a driver monitoring system. The method verifies proper hand placement and monitors driver health and correlates physiological responses with driving events, potentially enhancing both safety and driver wellbeing.

The various timing parameters (M seconds, K seconds) and thresholds can be adjusted based on specific implementation requirements and safety considerations. The system's ability to distinguish between different conditions (no hands, interferent objects, human hands, crossed hands) and respond appropriately demonstrates its sophistication and robustness for automotive safety applications. In embodiments, M and K are equal to ten seconds.

Figure 9 illustrates a controller system 900 that can be implemented as controller 208 in a hands-on detection system, according to an embodiment of the disclosure. The controller system 900 includes a processor 902, a memory 904, an interface 906, and a bus 908, which can be arranged as shown. Controller system 900 can include additional components not shown in this simplified block diagram.

Processor 902 can comprise various types of processing hardware circuitry capable of executing instructions for hands-on detection and ECG signal analysis operations. It can be implemented as a microcontroller, a Digital Signal Processor (DSP), an Application-Specific Integrated Circuit (ASIC), a Field-Programmable Gate Array (FPGA), or combinations thereof. In some implementations, processor 902 includes specialized circuits for analyzing ECG signals, performing morphology checks, and validating reference voltage patterns received through the electrodes positioned on the steering wheel.

Memory 904 can include non-transitory or transitory memory components. Non-transitory memory can include Read-Only Memory (ROM), Flash memory, or Electrically Erasable Programmable Read-Only Memory (EEPROM) for storing firmware or configuration parameters. Transitory memory can include Random Access Memory (RAM) such as Static RAM (SRAM) or other volatile memory types for temporary data storage during operation. Memory 904 can store operational parameters for hands-on detection and ECG analysis, including threshold values for signal amplitude verification, pulse width ranges, acceptable heart rate ranges, and program instructions implementing the methods described herein.

Interface 906 facilitates communication with the vertical analog front end (VAFE) circuit 206, allowing the controller system 900 to receive the electrostatic charge variation signals and amplified ECG signals from the electrodes on the steering wheel. Interface 906 can include analog-to-digital converters for sampling the biopotential signals, digital-to-analog converters for setting reference voltage patterns, and digital inputs/outputs for controlling the signal transmission and reception functions. The interface can also provide connectivity for external communications to enable integration with vehicle systems such as advanced driver assistance systems (ADAS) or dashboard displays for alerting the driver.

Bus 908 provides a communication pathway between processor 902, memory 904, and interface 906, enabling data transfer and coordination between these components during hands-on detection and ECG analysis operations. The bus 908 can comprise multiple signal lines for transferring data, addresses, and control signals.

In embodiments, the controller system 900 implements the hands-on detection and ECG verification methods by continuously monitoring signals from the steering wheel electrodes, analyzing the received patterns to verify physical contact, processing ECG signals to confirm valid cardiac activity, and making determinations about proper hand placement based on the combined analysis. The controller can also store heart rate data, correlate it with driving events detected through motion sensors, and generate appropriate alerts or recommendations based on the analysis results.

It should be appreciated that controller system 900 can be integrated within the same integrated circuit as the VAFE circuit 206 or can be implemented as a separate component. In some embodiments, the functionality of controller system 900 can be distributed across multiple components or integrated with other vehicle systems to optimize overall performance and efficiency. Depending on specific implementation requirements and integration considerations, the controller can be positioned within the steering wheel itself, in the steering column, or elsewhere in the vehicle.

A first aspect relates to a system for hands-on detection, the system comprising a plurality of electrodes positioned on a steering wheel; an analog front-end circuit coupled to the plurality of electrodes, the analog front-end circuit configured to: transmit a signal through at least one of the plurality of electrodes, receive the signal through at least one other of the plurality of electrodes in response to hands being in contact with the steering wheel, provide a reference voltage to at least one of the plurality of electrodes, and detect biopotential signals between at least two of the plurality of electrodes; and a controller coupled to the analog front-end circuit, the controller configured to: verify physical contact with the steering wheel based on the received signal, analyze the biopotential signals to verify presence of physiological characteristics, and generate a response based on the analysis of the biopotential signals.

In a first implementation form of the system, according to the first aspect as such, the controller is further configured to determine proper hand placement on the steering wheel based on the verified physical contact and the verified presence of physiological characteristics.

In a second implementation form of the system, according to the first aspect as such or any preceding implementation form of the first aspect, the plurality of electrodes comprises a pair of receiving electrodes coupled to the analog front-end circuit for receiving the signal and detecting the biopotential signals; a pair of stimulus electrodes coupled to the analog front-end circuit for transmitting the signal; and a pair of reference electrodes coupled to the analog front-end circuit for providing the reference voltage.

In a third implementation form of the system, according to the first aspect as such or any preceding implementation form of the first aspect, the pair of reference electrodes are positioned adjacent to the pair of receiving electrodes to provide local polarization that establishes a constant bias for filtering out environmental electrical noise.

In a fourth implementation form of the system, according to the first aspect as such or any preceding implementation form of the first aspect, the analog front-end circuit comprises a vertical analog front end circuit that includes differential inputs coupled to at least two of the plurality of electrodes for measuring a potential difference between the differential inputs.

In a fifth implementation form of the system, according to the first aspect as such or any preceding implementation form of the first aspect, the biopotential signals comprise electrocardiogram (ECG) signals, and wherein the controller is configured to analyze the ECG signals to verify that the ECG signals represent valid cardiac activity.

In a sixth implementation form of the system, according to the first aspect as such or any preceding implementation form of the first aspect, analyzing the ECG signals comprises performing a morphology check that examines signal amplitude, peak width, beat-to-beat intervals, or a combination thereof.

A second aspect relates to a method for hands-on detection on a steering wheel, the method comprising: transmitting a reference voltage pattern through stimulus electrodes positioned on the steering wheel; receiving an electrostatic charge variation signal through receiving electrodes positioned on the steering wheel in response to hands bridging the stimulus electrodes and the receiving electrodes; providing a reference voltage to a reference electrodes positioned adjacent to the receiving electrodes to establish local polarization; detecting electrocardiogram (ECG) signals between the receiving electrodes positioned on opposite sides of the steering wheel; verifying physical contact with the steering wheel by validating the voltage pattern through the electrostatic charge variation signal; analyzing the detected ECG signals to verify presence of physiological characteristics; and generating a response based on the analysis of the detected ECG signals.

In a first implementation form of the method, according to the second aspect as such, the method further comprising determining that both hands are properly positioned on the steering wheel based on the validated reference voltage pattern and the verified presence of physiological characteristics.

In a second implementation form of the method, according to the second aspect as such or any preceding implementation form of the second aspect, validating the reference voltage pattern comprises sampling the electrostatic charge variation signal at specific intervals; applying filtering algorithms to remove noise; decoding the filtered signal to recover a bit pattern; and comparing the recovered bit pattern with an expected pattern.

In a third implementation form of the method, according to the second aspect as such or any preceding implementation form of the second aspect, analyzing the detected ECG signals comprises: performing a morphology check on waveform characteristics of the detected ECG signals; examining features including P waves, QRS complexes, T waves, or a combination thereof; and verifying that intervals between successive waves fall within expected ranges.

In a fourth implementation form of the method, according to the second aspect as such or any preceding implementation form of the second aspect, the method further comprising scanning multiple electrode sets distributed around a circumference of the steering wheel; detecting hand contact at multiple positions through the scanning; and determining specific hand positions on the steering wheel.

In a fifth implementation form of the method, according to the second aspect as such or any preceding implementation form of the second aspect, the method further comprising detecting crossed hands by analyzing QRS complex inversion in the ECG signals.

In a sixth implementation form of the method, according to the second aspect as such or any preceding implementation form of the second aspect, the method further comprising collecting motion sensor data from accelerometers and gyroscopes; identifying specific driving maneuvers based on the motion sensor data; and correlating heart rate information with the identified driving maneuvers.

A third aspect relates to a system, the system comprising a steering wheel with a plurality of electrode sets distributed around its circumference, each electrode set comprising a receiving electrode, a stimulus electrode, and a reference electrode; a vertical analog front end circuit coupled to the multiple electrode sets, the vertical analog front end circuit configured to: transmit reference voltage patterns through the stimulus electrodes, detect electrostatic charge variations through the receiving electrodes, and provide reference voltages to the reference electrodes for local polarization; and a controller coupled to the vertical analog front end circuit, the controller configured to scan the multiple electrode sets to detect hand contact at various positions around the steering wheel, analyze detected signals to verify presence of physiological characteristics, generate a response based on the analysis of the detected signals, monitor heart rate information derived from the detected signals, and correlate information on the heart rate with driving maneuvers detected through motion sensors.

In a first implementation form of the system, according to the third aspect as such, the controller is further configured to determine hand positions on the steering wheel based on the scanning and the verified presence of physiological characteristics.

In a second implementation form of the system, according to the third aspect as such or any preceding implementation form of the third aspect, generating a response based on the analysis of the detected signals comprises providing a visual alert on a dashboard display; providing an audible alert to the driver; disabling a set of vehicle functions in response to improper hand placement; enabling advanced driver assistance features in response to proper hand placement; recording health monitoring data based on the verified physiological characteristics, or a combination thereof.

In a third implementation form of the system, according to the third aspect as such or any preceding implementation form of the third aspect, the controller is configured to detect crossed hands by analyzing polarity inversion in the detected signals.

In a fourth implementation form of the system, according to the third aspect as such or any preceding implementation form of the third aspect, the controller is further configured to generate a signal to the driver in response to detecting an interferent between the driver's hands and the steering wheel, the signal indicating to remove the interferent.

In a fifth implementation form of the system, according to the third aspect as such or any preceding implementation form of the third aspect, the controller implements an overlapping window approach that analyzes at least three peaks of a cardiac cycle to determine the heart rate.

Although the description has been described in detail, it should be understood that various changes, substitutions, and alterations can be made without departing from the spirit and scope of this disclosure as defined by the appended claims. The same elements are designated with the same reference numbers in the various figures. Moreover, the scope of the disclosure is not intended to be limited to the particular embodiments described herein, as one of ordinary skill in the art will readily appreciate from this disclosure that processes, machines, manufacture, compositions of matter, means, methods, or steps, presently existing or later to be developed, perform substantially the same function or achieve substantially the same result as the corresponding embodiments described herein. Accordingly, the appended claims are intended to include within their scope such processes, machines, manufacture, compositions of matter, means, methods, or steps.

The specification and drawings are, accordingly, to be regarded simply as an illustration of the disclosure as defined by the appended claims, and are contemplated to cover any and all modifications, variations, combinations, or equivalents that fall within the scope of the present disclosure.

## Claims

1. A system for hands-on detection, the system comprising:
- a plurality of electrodes (202a, 202b, 204a, 204b, 502a, 502b) positioned on a steering wheel;
- an analog front-end circuit (206) coupled to the plurality of electrodes, the analog front-end circuit configured to:
transmit a signal through at least one of the plurality of electrodes,
receive the signal through at least one other of the plurality of electrodes in response to hands (104) being in contact with the steering wheel (102),
provide a reference voltage to at least one of the plurality of electrodes, and
detect biopotential signals between at least two of the plurality of electrodes (202a, 202b); and
- a controller (208) coupled to the analog front-end circuit (206), the controller configured to:
verify physical contact with the steering wheel (102) based on the received signal,
analyze the biopotential signals to verify presence of physiological characteristics, and
generate a response based on the analysis of the biopotential signals.

2. The system of claim 1, wherein the controller (208) is further configured to determine hand placement on the steering wheel (102) based on the verified physical contact and the verified presence of physiological characteristics.

3. The system of claim 1, wherein the plurality of electrodes comprises:
a pair of receiving electrodes (202a, 202b) coupled to the analog front-end circuit (206) for receiving the signal and detecting the biopotential signals;
a pair of stimulus electrodes (502a, 502b) coupled to the analog front-end circuit (206) for transmitting the signal; and
a pair of reference electrodes (204a, 204b) coupled to the analog front-end circuit (206) for providing the reference voltage.

4. The system of claim 3, wherein the pair of reference electrodes (204a, 204b) are positioned adjacent to the pair of receiving electrodes (202a, 202b) to provide local polarization that establishes a constant bias for filtering out environmental electrical noise.

5. The system of claim 1, wherein the analog front-end circuit (206) comprises a vertical analog front end circuit that includes differential inputs (Q+, Q-) coupled to at least two of the plurality of electrodes (202a, 202b) for measuring a potential difference between the differential inputs.

6. The system of claim 1, wherein the biopotential signals comprise electrocardiogram, ECG, signals, and wherein the controller (208) is configured to analyze the ECG signals to verify that the ECG signals represent valid cardiac activity.

7. The system of claim 6, wherein analyzing the ECG signals comprises performing a morphology check that examines signal amplitude, peak width, beat-to-beat intervals, or a combination thereof.

8. The system of claim 1, wherein the plurality of electrodes comprises a plurality of electrode sets (202a, 204a, 502a, 202b, 204b, 502b) distributed around the circumference of the steering wheel (102), and wherein the controller (208) is further configured to:
scan the electrode sets to detect hand contact at various positions around the steering wheel,
monitor heart rate information derived from the detected signals, and
correlate information on the heart rate with driving maneuvers detected through motion sensors.

9. The system of claim 8, wherein generating a response based on the analysis of the detected signals comprises providing a visual alert on a dashboard display, providing an audible alert to a driver of vehicle hosting the system, disabling a set of vehicle functions in response to improper hand placement, enabling advanced driver assistance features in response to proper hand placement, recording health monitoring data based on the verified physiological characteristics, or a combination thereof.

10. The system of claim 8 or 9, wherein the controller (208) is configured to detect crossed hands by analyzing polarity inversion in the detected signals.

11. The system of anyone of claims 8-10, wherein the controller (208) is further configured to generate a signal to a driver of vehicle hosting the system in response to detecting an interferent between hands (104) of the driver and the steering wheel (102), the signal indicating to remove the interferent.

12. The system of anyone of claims 8-11, wherein the controller (208) implements an overlapping window approach that analyzes at least three peaks of a cardiac cycle to determine the heart rate.

13. A method for hands-on detection on a steering wheel (102), the method comprising:
transmitting a reference voltage pattern through stimulus electrodes (502a, 502b) positioned on the steering wheel;
receiving an electrostatic charge variation signal through receiving electrodes (202a, 202b) positioned on the steering wheel in response to hands (104) bridging the stimulus electrodes and the receiving electrodes;
providing a reference voltage to a reference electrodes (204a, 204b) positioned adjacent to the receiving electrodes to establish local polarization;
detecting electrocardiogram, ECG, signals between the receiving electrodes positioned on opposite sides of the steering wheel;
verifying physical contact with the steering wheel by validating the voltage pattern through the electrostatic charge variation signal;
analyzing the detected ECG signals to verify presence of physiological characteristics; and
generating a response based on the analysis of the detected ECG signals.

14. The method of claim 13, wherein validating the reference voltage pattern comprises:
sampling the electrostatic charge variation signal at specific intervals;
applying filtering algorithms to remove noise;
decoding the filtered signal to recover a bit pattern; and
comparing the recovered bit pattern with an expected pattern.

15. The method of claim 13 or claim 14, wherein analyzing the detected ECG signals comprises:
performing a morphology check on waveform characteristics of the detected ECG signals;
examining features including P waves, QRS complexes, T waves, or a combination thereof; and
verifying that intervals between successive waves fall within expected ranges.
